(19) Europäisches Patentamt
European Patent Office
Office européen des brevets

(11) Veröffentlichungsnummer: **0 030 923**
**A1**

(12) **EUROPÄISCHE PATENTANMELDUNG**

(21) Anmeldenummer: **80810385.7**

(22) Anmeldetag: **12.12.80**

(51) Int. Cl.³: **C 07 D 263/26**
C 07 D 413/12, A 01 N 43/76
//C07C133/02

(30) Priorität: **18.12.79 CH 11215/79**

(43) Veröffentlichungstag der Anmeldung:
**24.06.81 Patentblatt 81/25**

(84) Benannte Vertragsstaaten:
**CH DE FR IT LI**

(71) Anmelder: **CIBA-GEIGY AG**
**Patentabteilung Postfach**
**CH-4002 Basel(CH)**

(72) Erfinder: **Eckhardt, Wolfgang, Dr.**
**Breslauerstrasse 16**
**D-7850 Lörrach(DE)**

(72) Erfinder: **Kunz, Walter, Dr.**
**Im Goldbrunnen 55**
**CH-4104 Oberwil(CH)**

(72) Erfinder: **Hubele, Adolf, Dr.**
**Obere Egg 9**
**CH-4465 Magden(CH)**

(54) **3-(N-Acyl-phenylamino)-oxazolidin-2-one, Verfahren zu ihrer Herstellung sowie deren Verwendung als Mikrobizide oder in mikrobiziden Mitteln.**

(57) Verbindungen der Formel I

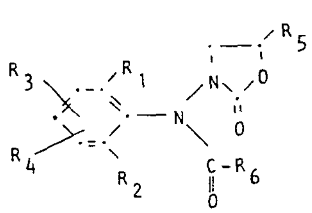

(1)

worin
$R_1$ $C_1$-$C_3$-Alkyl, $C_1$-$C_3$-Alkoxy oder Halogen,
$R_2$ $C_1$-$C_3$-Alkyl, Halogen oder Nitro,
$R_3$ Wasserstoff, Halogen, Nitro oder Methyl,
$R_4$ Wasserstoff oder Methyl und
$R_6$ für $-CH_2OR_7$, 1H-1,2,4-Triazolylmethyl, Halogenmethyl, Cyclopropyl, Furyl, Tetrahydrofuryl, $-CH_2N(C_1$-$C_3$-Alkyl$)_2$, $-CH_2NHN(R_8)(R_9)$ oder $-CH_2S(O)_n$ ($C_1$-$C_3$-Alkyl) steht, wobei
$R_7$ Wasserstoff, $-SO_2(C_1$-$C_3$-Alkyl), $-SO_2NHCH_3$, Acetyl oder $C_1$-$C_3$-Alkyl,
$R_8$ Wasserstoff oder $C_1$-$C_3$-Alkyl,
$R_9$ $C_1$-$C_3$-Alkyl oder Phenyl bedeuten und
n 0, 1 oder 2 darstellt.
Ihre Herstellung und ihre Verwendung zur Bekämpfung pflanzenschädlicher Mikro-organismen, insbesondere gegen phytopatogene Pilze, und Bakterien.

EP 0 030 923 A1

CIBA-GEIGY AG
Basel (Schweiz)

5-12643/-

# 3-(N-Acyl-phenylamino)-oxazolidin-2-one, Verfahren zu ihrer Herstellung sowie deren Verwendung als Mikrobizide oder in mikrobiziden Mitteln.

Die vorliegende Erfindung betrifft Verbindungen der Formel I

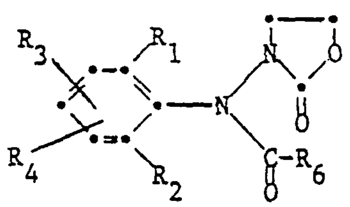

(I)

worin

$R_1$ $C_1$-$C_3$-Alkyl, $C_1$-$C_3$-Alkoxy oder Halogen,
$R_2$ $C_1$-$C_3$-Alkyl, Halogen oder Nitro,
$R_3$ Wasserstoff, Halogen, Nitro oder Methyl,
$R_4$ Wasserstoff oder Methyl und
$R_6$ für $-CH_2OR_7$, 1H-1,2,4-Triazolylmethyl, Halogenmethyl, Cyclopropyl, Furyl, Tetrahydrofuryl, $-CH_2N(C_1-C_3\text{-Alkyl})_2$, $-CH_2NHN(R_8)(R_9)$ oder $-CH_2S(O)_n(C_1-C_3\text{-Alkyl})$ steht, wobei
$R_7$ Wasserstoff, $-SO_2(C_1-C_3\text{-Alkyl})$, $-SO_2NHCH_3$, Acetyl oder $C_1$-$C_3$-Alkyl,
$R_8$ Wasserstoff oder $C_1$-$C_3$-Alkyl,
$R_9$ $C_1$-$C_3$-Alkyl oder Phenyl bedeuten und
n 0, 1 oder 2 darstellt.

Unter Alkyl oder Alkylanteil eines anderen Substituenten sind Methyl, Ethyl, Propyl und Isopropyl zu verstehen. Unter

Halogen soll hier und im folgenden Fluor, Chlor, Brom oder Jod verstanden werden.

Verbindungen der Formel I sind bei Raumtemperatur luftstabile Substanzen, sie zeigen ein sehr wertvolles Mikrobizid-Spektrum und wirken insbesondere gegen phytopathogene Mikroorganismen, vor allem gegen pflanzenschädigende Pilze.

Die nachfolgenden Substituententypen oder Kombinationen dieser untereinander werden bevorzugt.

Bei $R_1$: -a) $C_1$-$C_3$-Alkyl, $C_1$-$C_3$-Alkoxy, Halogen
-b) Methyl, Methoxy, Chlor

Bei $R_2$: -a) $C_1$-$C_3$-Alkyl, Halogen
-b) Methyl, Chlor

Bei $R_3$: -a) Wasserstoff, Methyl, Halogen, Nitro
-b) Wasserstoff, Methyl, Chlor

Bei $R_4$: Wasserstoff, Methyl

Bei $R_6$: -a) $-CH_2OR_7$, 1H-1,2,4-Triazolylmethyl, $-CH_2Cl$, Tetrahydrofuryl, $-CH_2S-(C_1-C_3\text{-Alkyl})$
-b) $-CH_2OR_7$, 1H-1,2,4-Triazolylmethyl, 2-Tetrahydrofuryl
-c) $-CH_2OR_7$

Bei $R_7$: -a) $C_1$-$C_3$-Alkyl, $-SO_2-(C_1-C_3\text{-Alkyl})$, $-SO_2NHCH_3$
-b) $C_1$-$C_3$-Alkyl, $-SO_2CH_3$, $-SO_2NHCH_3$

Es ergeben sich somit z.B. folgende bevorzugte Gruppen:

Eine bevorzugte Gruppe von Mikrobiziden bilden Verbindungen der Formel I, worin $R_1$ für $C_1$-$C_3$-Alkyl, $C_1$-$C_3$-Alkoxy oder Halogen, $R_2$ für $C_1$-$C_3$-Alkyl oder Halogen, $R_3$ für Wasserstoff, Methyl, Halogen, oder Nitro, $R_4$ für Wasserstoff oder Methyl stehen und $R_6$ $-CH_2OR_7$, 1H-1,2,4-Triazolylmethyl, Chlormethyl, Tetrahydrofuryl oder $-CH_2S(C_1$-$C_3$-Alkyl) bedeutet, wobei $R_7$ für $C_1$-$C_3$-Alkyl, $-SO_2(C_1$-$C_3$-Alkyl) oder $-SO_2NHCH_3$ steht.

Eine weitere bevorzugte Gruppe von Mikrobiziden bilden Verbindungen der Formel I, worin $R_6$ für $-CH_2OR_7$, 1H-1,2,4-Triazolylmethyl oder 2-Tetrahydrofuryl steht, wobei $R_7$ für $C_1$-$C_3$-Alkyl, $-SO_2(C_1$-$C_3$-Alkyl) oder $-SO_2NHCH_3$ steht.

Eine besonders bevorzugte Gruppe von Mikrobiziden bilden Verbindungen der Formel I, worin $R_1$ Methyl, Methoxy oder Chlor, $R_2$ Methyl oder Chlor, $R_3$ Wasserstoff, Methyl oder Chlor, $R_4$ Wasserstoff oder Methyl bedeuten und $R_6$ für $-CH_2OR_7$ steht wobei $R_7$ $C_1$-$C_3$-Alkyl, $-SO_2CH_3$ oder $-SO_2NHCH_3$ bedeutet.

Besonders bevorzugt werden die Verbindungen:

3-[(N-Methoxyacetyl)-2,6-dimethyl-phenylamino]-oxazolidin-2-on und
3-[(N-Methoxyacetyl)-2-chlor-6-methyl-phenylamino]-oxazolidin-2-on.

Die Verbindungen der Formel I werden erfindungsgemäss aus Verbindungen der Formel II

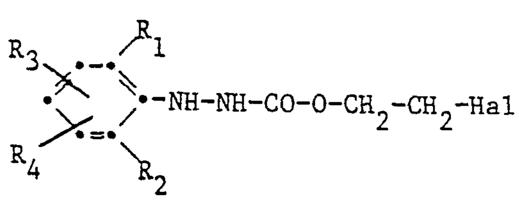

(II)

hergestellt, worin $R_1$, $R_2$, $R_3$ und $R_4$ die unter Formel I angegebenen Bedeutungen haben und Hal für Halogen steht, indem man, in der Reihenfolge wahlweise a) mit einer Säure der Formel $HOOCR_6$ bzw. einem ihrer reaktionsfähigen Derivate acyliert, wobei $R_6$ die unter Formel I angegebenen Bedeutungen hat und b) in alkalischem Medium durch intramolekulare Halogenwasserstoff-Eliminierung cyclisiert und, falls $R_6$ für Halogenmethyl steht, die erhaltenen Verbindungen, sofern gewünscht, wahlweise mit einer der Verbindungen der Formel III, IV, V oder VI

$HN(C_1\text{-}C_3\text{-Alkyl})_2$ (III)

$H_2N\text{-}N(R_8)(R_9)$ (IV)

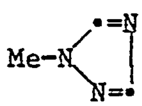
(V)

$MeOR_7$ (VI)

reagieren lässt, wobei $R_7$, $R_8$, $R_9$ und X die unter Formel I angegebenen Bedeutungen haben und Me für Wasserstoff oder ein Metallkation steht.

Zur N-Acylierung von Verbindungen der Formel II bzw. VII

(II)
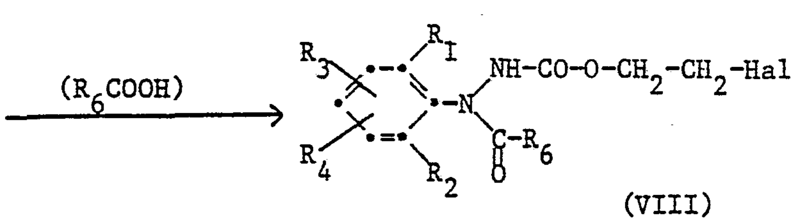

(VIII)

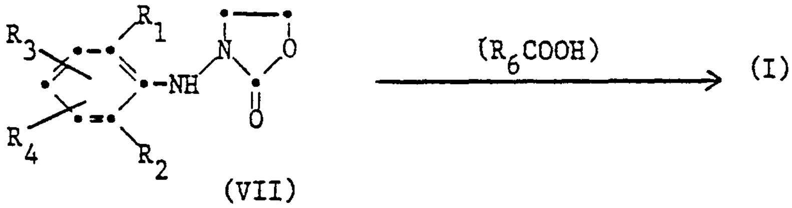

(VII)

zu Zwischenprodukten der Formel VIII bzw. Endprodukten I lässt sich die entsprechende Carbonsäure der Formel $R_6COOH$ selbst sowie ihre Ester, vorteilhaft jedoch ihr Säureanhydrid oder eines ihrer Säurehalogenide, bevorzugt das Säurechlorid oder Säurebromid einsetzen.

Die Reaktionstemperaturen liegen zwischen 0° und 180°C, vorzugsweise 0° und 150°C bzw. am Siedepunkt des Lösungsmittels bzw. Lösungsmittelgemisches. In manchen Fällen ist die Verwendung von säurebindenden Mitteln bzw. Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Entstehender Halogenwasserstoff kann in manchen Fällen auch mittels Durchleiten von Inertgas, z.B. Stickstoff aus dem Reaktionsgemisch vertrieben werden.

Die N-Acylierung kann in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid;

Dimethylsulfoxid; Ketone wie Aceton, Diethylketon, Methylethylketon und Gemische solcher Lösungsmittel untereinander. In manchen Fällen kann das Acylierungsmittel selbst als Lösungsmittel dienen.

Bei der Acylierung kann die Gegenwart eines Reaktionskatalysators wie Dimethylformamid von Vorteil sein.

Die Cyclisierung von Verbindungen der Formel II bzw. VIII zu Zwischenprodukten der Formel VII bzw. Endprodukten I wird vorteilhaft unter Eliminierung von Halogenwasserstoff in alkalischem Medium durchgeführt. Hierbei liegen die Reaktionstemperaturen zwischen 0° und 150°C, vorzugsweise 0° bis 100°C. Man verwendet zweckmässigerweise wasserfreie reaktionsinerte Lösungsmittel wie aliphatische oder aromatische Kohlenwasserstoffe; insbesondere eignen sich hierzu Alkanole wie Methanol, Ethanol, Propanol usw. oder Gemische solcher Lösungsmittel. Die Reaktion wird vorteilhafterweise in Gegenwart einer starken Base z.B. Alkali- oder Erdalkalihydroxid oder -carbonat oder Alkalimetall durchgeführt, wobei in manchen Fällen das Arbeiten unter Schutzgasatmosphäre z.B. Stickstoff von Vorteil sein kann.

Aus Verbindungen der Formel I, worin $R_6$ für Halomethyl ($-CH_2Cl$, $-CH_2Br$, $-CH_2J$) steht, lassen sich weitere Endprodukte der Formel I herstellen, z.B. durch Reaktion mit einer der Verbindungen der Formel III bis VI unter Abspaltung von Halogenwasserstoff bzw. Alkalisalz des entsprechenden Halogens. In den Formeln V und VI steht Me für Wasserstoff oder ein Metallkation, vorzugsweise ein Alkalimetallkation. Die Reaktion wird zweckmässigerweise in einem reaktionsinerten, organischen Lösungsmittel durchgeführt. Bevorzugt werden relativ polare, jedoch inerte Lösungsmittel wie N,N-Dimethylformamid, N,N-Dimethylacetamid, Dimethylsulfoxid, Acetonitril, Benzonitril und andere. Diese Lösungsmittel können mit weiteren, in der organischen Chemie üblichen, inerten Lösungsmitteln wie aliphatischen und aromatischen Kohlenwasserstoffen, z.B. Benzol, Toluol, Xylol, Hexan, Petrolether, Chlorbenzol, Nitrobenzol usw. verwendet werden.

Zur Beschleunigung der Reaktionsgeschwindigkeit kann ein Alkalihalogenid, insbesondere -Jodid wie NaJ oder KJ zugesetzt werden.

Man kann das Verfahren zweckmässigerweise auch in Gegenwart einer Base durchführen. Als Basen kommen organische und anorganische Basen in Frage, z.B. Oxide, Hydroxide, Hydride, Carbonate und Hydrogencarbonate von Alkali- und Erdalkalimetallen sowie tertiäre Amine wie Trialkylamin, Pyridin und Pyridinbasen.

Die Reaktionstemperaturen liegen hierbei zwischen 0° und 150°C bzw. am Siedepunkt des Lösungsmittels bzw. Lösungsmittelgemisches. Ueblicherweise arbeitet man jedoch bei Raumtemperatur.

Auch aus Verbindungen der Formel I, worin $R_6$ für Hydroxymethyl steht, lassen sich weitere Endprodukte der Formel I herstellen, dabei kann die Hydroxymethylgruppe zweckmässigerweise durch Veresterung der Halomethylgruppe (z.B. mit $CH_3COONa$) und anschliessender alkalischer Hydrolyse der Estergruppe hergestellt werden.

Verbindungen der Formel I, worin $R_6$ für Hydroxymethyl steht reagieren mit Säurehalogeniden, vorzugsweise Chloriden oder Bromiden der Formel Hal-$SO_2$($C_1$-$C_3$-Alkyl) oder Hal-$SO_2NHCH_3$ zu den entsprechend substituierten Produkten der Formel I. Es eignen sich hierzu die gleichen Reaktionsbedingungen, wie bei den beschriebenen Acylierungsreaktionen.

Die Ausgangsverbindungen der Formel II lassen sich aus den zugrundeliegenden Phenylhydrazinen, in Form eines Hydrohalogenids IX

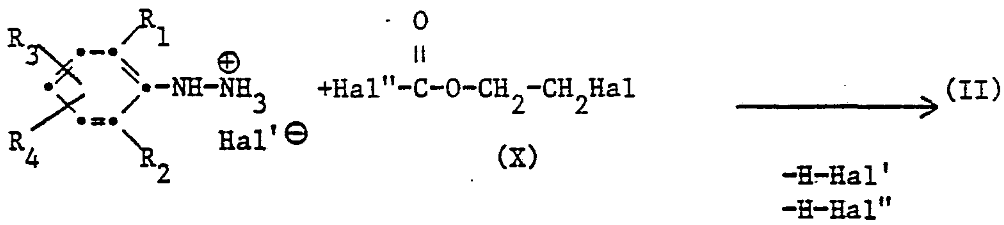


(IX) (X) (II)

durch Reaktion mit dem gewünschten Halogenameisensäureester der Formel X, wobei $R_1$, $R_2$, $R_3$ und $R_4$ die unter Formel I angegebenen Bedeutungen hat und Hal, Hal' sowie Hal" für Halogen (Chlor, Brom, Jod) stehen.

Diese Reaktion kann analog zu den bereits beschriebenen Acylierungsreaktionen durchgeführt werden.

Das Herstellungsverfahren von Verbindungen der Formel I ist einschliesslich aller beschriebenen Varianten ein Bestandteil der Erfindung.

Die Ausgangsverbindungen der Formel IX und X sind bekannt und lassen sich nach an sich bekannten Methoden herstellen.

Die Zwischenprodukte der Formel VIII sind neu und lassen sich nach dem hierin beschriebenen Verfahren herstellen. Sie zeigen überraschenderweise eine gute Wirkung gegen schädliche Mikroorganismen, insbesondere gegen phytopathogene Pilze und können zum Schutz von Kulturpflanzen angewendet werden.

Die Verbindungen der Formel I und der Formel VIII sind luftstabile Substanzen, die sich in den meisten üblichen organischen Solventien lösen. Sie lassen sich durch starke Säuren und Laugen, insbesondere bei erhöhter Temperatur zerstören.

Herstellung und chemische Eigenschaften von 3-(N-Acetyl-N-phenylamino)-oxazolidin-2-on werden beschrieben in J. Org. Chem. 31 No. 3, S. 968-970 (1966). Eine biologische Wirkung dieser Verbindung wird nicht berichtet.

Es wurde überraschend gefunden, dass Verbindungen der Formel I ein für praktische Bedürfnisse sehr günstiges Mikrobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative und präventive Wirkung zum Schutz von Kulturpflanzen ohne diese durch unerwünschte Nebenwirkungen zu beeinflussen. Kulturpflanzen seien im Rahmen vorliegender Erfindung beispielsweise: Getreide: (Weizen, Gerste, Roggen, Hafer, Reis); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Wirkstoffe der Formel I sind unter anderem gegen folgende phytopathogene Pilze wirksam: z.B. gegen die zu der Familie der Phycomycetes gehörenden Oomycetes wie Phytophthora, Peronospora und Pythium sowie gegen Fungi imperfecti wie Botrytis. Darüberhinaus wirken Verbindungen der Formel I auch bakterizid so z.B. gegen die zur Familie der Pseudomonadaceae gehörenden Xanthomonas.

Sie können ferner als Beizmittel zur Behandlung von Saatgut (Früchte, Knollen, Körner) und Pflanzenstecklingen zum Schutz vor Pilzinfektionen sowie gegen im Erdboden auftretende Mikroorganismen eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Zur Bekämpfung dieser Mikroorganismen können die Verbindungen der Formel I für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Dispergier-, Netz-, Haft-, Verdickungs-, Binde- oder Düngemitteln. Wirkstoffe der Formel I können auch im Gemisch mit z.B. pestiziden oder pflanzenwuchsverbessernden Präparaten verwendet werden.

Der Gehalt an Wirkstoff liegt dabei in handelsfähigen Mitteln im allgemeinen zwischen 0,0001 und 90%.

In den nachfolgenden Beispielen sind Temperaturen in Celsius-Graden angegeben, Teile bedeuten Gewichtsteile. Prozentangaben beziehen sich stets auf Gewicht.

Herstellungsbeispiele

Beispiel 1: a) Herstellung des Zwischenproduktes

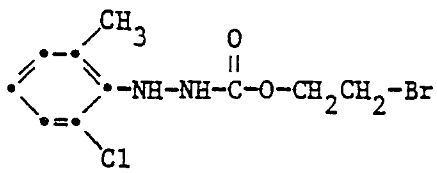

3-(2-Chlor-6-methyl-phenyl)-(2-bromethyl)-carbazat

Zu einer Lösung von 16,8 g (0,3 Mol) Kaliumhydroxid und 29 g (0,15 Mol) 2-Chlor-6-methyl-phenyl-hydrazoniumhydrochlorid in 300 ml Wasser lässt man bei Raumtemperatur und unter Rühren 28,1 g (0,15 Mol) Chlorameisensäure-2-brom-ethylacetat in 50 ml Tetrahydrofuran tropfen und rührt noch 1 Stunde bei Raumtemperatur weiter. Das sich abscheidende Oel wird in Diethylether aufgenommen, mit 2-normaler wässriger Natriumhydroxidlösung gewaschen, über Natriumsulfat getrocknet, filtriert und eingeengt. Nach Zugabe von Petrolether erhält man 18 g der kristallinen Verbindung. Smp. 53-61°.

b) Herstellung des Zwischenproduktes

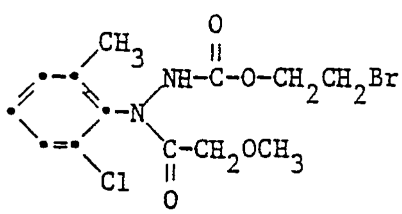

(2.8)

3-Methoxyacetyl-3-(2-chlor-6-methyl-phenyl)-(2-bromethyl)-carbazat

Zu einer Lösung von 15 g (0,05 Mol) 3-(-2-Chlor-6-methyl-phenyl)-[(-2-bromethyl)-carbazat] in 150 ml wasserfreiem Toluol lässt man bei Raumtemperatur 6 g (0,055 Mol) Methoxyacetylchlorid in 50 ml Toluol tropfen. Das Gemisch wird unter Einleitung von Stickstoff 16 Stunden unter Rückfluss erhitzt. Nach dem Einengen wird der Rückstand aus Petrolether/Diethylether umkristallisiert. Ausbeute 14 g. Smp. 127-129°.

c) Herstellung des Endproduktes

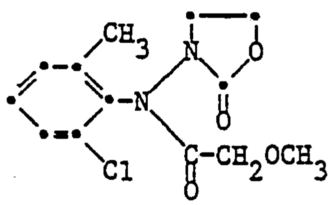

(1.24)

3-[(N-Methoxyacetyl)-2-chlor-6-methyl-phenylamino]-oxazolidin-2-on

9,5 g (0,025 Mol) 3-Methoxyacetyl-3-(-2-chlor-6-methyl-phenyl)—(-2-bromethyl)-carbazat und 1,7 g (0,025 Mol) Kaliumhydroxid werden 40 Stunden bei Raumtemperatur in 150 ml absolutem Ethanol gerührt. Nach dem Filtrieren und Einengen der Lösung wird der Rückstand in Methylenchlorid aufgenommen, mit Wasser gewaschen, über Natriumsulfat getrocknet, filtriert und eingeengt. Das zurückbleibende Oel wird durch Zugabe von Petrolether zur Kristallisation gebracht. Ausbeute 6,5 g. Smp. 86-90°.

Auf analoge Weise werden folgende Endprodukte der Formel I und Zwischenprodukte der Formel VIII hergestellt:

(Tabellen 1 und 2: 1-H-Triazolylmethyl = 1-H-1,2,4-Triazolylmethyl)

Tabelle 1: Verbindungen der Formel I

| Verb. Nr. | $R_1$ | $R_2$ | $R_3$ | $R_4$ | $R_6$ | Physik. Konst. |
|---|---|---|---|---|---|---|
| 1.1 | $CH_3$ | $CH_3$ | H | H | $-CH_2OCH_3$ | Oel |
| 1.2 | $CH_3$ | $CH_3$ | H | H | Cyclopropyl | |
| 1.3 | $CH_3$ | $CH_3$ | H | H | $-CH_2Cl$ | Oel |
| 1.4 | $CH_3$ | $CH_3$ | H | H | 2-Tetrahydrofuryl | |
| 1.5 | $CH_3$ | $CH_3$ | H | H | 2-Furyl | |
| 1.6 | $CH_3$ | $CH_3$ | H | H | $-CH_2SCH_3$ | |
| 1.7 | $CH_3$ | $CH_3$ | H | H | $-CH_2SO_2CH_3$ | |
| 1.8 | $CH_3$ | $CH_3$ | H | H | 1H-Triazolylmethyl | Smp.53-56°C |
| 1.9 | $CH_3$ | $CH_3$ | H | H | $-CH_2NHN(CH_3)_2$ | |
| 1.10 | $CH_3$ | $CH_3$ | H | H | $-CH_2N(CH_3)_2$ | |
| 1.11 | $CH_3$ | $CH_3$ | H | H | $-CH_2OH$ | |
| 1.12 | $CH_3$ | $CH_3$ | H | H | $-CH_2OCOCH_3$ | Oel |
| 1.13 | $CH_3$ | $CH_3$ | H | H | $-CH_2OSO_2CH_3$ | |
| 1.14 | $CH_3$ | $CH_3$ | H | H | $-CH_2OSO_2NHCH_3$ | |
| 1.15 | $CH_3$ | $CH_3$ | $3-CH_3$ | H | $-CH_2OCH_3$ | |
| 1.16 | $CH_3$ | $CH_3$ | $3-CH_3$ | H | Cyclopropyl | |
| 1.17 | $CH_3$ | $CH_3$ | $3-CH_3$ | H | 2-Tetrahydrofuryl | |
| 1.18 | $CH_3$ | $CH_3$ | $2-CH_3$ | H | $-CH_2SCH_3$ | |
| 1.19 | $CH_3$ | $CH_3$ | $3-CH_3$ | H | $-CH_2Cl$ | |
| 1.20 | $CH_3$ | $CH_3$ | $3-CH_3$ | $5-CH_3$ | $-CH_2OCH_3$ | |
| 1.21 | $CH_3$ | $CH_3$ | $3-CH_3$ | $5-CH_3$ | Cyclopropyl | |
| 1.22 | $CH_3$ | $CH_3$ | $3-CH_3$ | $5-CH_3$ | $-CH_2OH$ | |

| Verb. Nr. | $R_1$ | $R_2$ | $R_3$ | $R_4$ | $R_6$ | Physik. Konst. |
|---|---|---|---|---|---|---|
| 1.23 | $CH_3$ | $CH_3$ | $3-CH_3$ | $5-CH_3$ | $-CH_2OSO_2NHCH_3$ | |
| 1.24 | $CH_3$ | Cl | H | H | $-CH_2OCH_3$ | Smp. 86-90°C |
| 1.25 | $CH_3$ | Cl | H | H | Cyclopropyl | |
| 1.26 | $CH_3$ | Cl | H | H | $-CH_2Cl$ | |
| 1.27 | $CH_3$ | Cl | H | H | $-CH_2OCOCH_3$ | |
| 1.28 | $CH_3$ | Cl | H | H | $-CH_2OH$ | |
| 1.29 | $CH_3$ | Cl | H | H | $-CH_2OSO_2CH_3$ | |
| 1.30 | $CH_3$ | Cl | H | H | $-CH_2OSO_2NHCH_3$ | |
| 1.31 | $CH_3$ | Cl | H | H | 1H-Triazolylmethyl | |
| 1.32 | $OCH_3$ | $CH_3$ | H | H | $-CH_2OCH_3$ | |
| 1.33 | $OCH_3$ | $CH_3$ | H | H | Cyclopropyl | |
| 1.34 | $CH_3$ | $NO_2$ | H | H | $-CH_2OCH_3$ | |
| 1.35 | $CH_3$ | $NO_2$ | H | H | $-CH_2OC_2H_5$ | |
| 1.36 | $CH_3$ | $NO_2$ | H | H | Cyclopropyl | |
| 1.37 | $CH_3$ | $CH_3$ | 3-Cl | H | $-CH_2OCH_3$ | |
| 1.38 | $CH_3$ | $CH_3$ | 3-Cl | H | Cyclopropyl | |
| 1.39 | $CH_3$ | $CH_3$ | 3-Cl | H | 1H-Triazolylmethyl | |
| 1.40 | $CH_3$ | $CH_3$ | 3-Cl | H | 2-Tetrahydrofuryl | |
| 1.41 | $CH_3$ | $CH_3$ | $3-NO_2$ | H | $-CH_2OCH_3$ | |
| 1.42 | $CH_3$ | $C_2H_5$ | H | H | 3-Furyl | |
| 1.43 | $CH_3$ | $C_2H_5$ | H | H | Cyclopropyl | |
| 1.44 | $CH_3$ | $C_2H_5$ | H | H | $-CH_2Cl$ | |
| 1.45 | $OCH_3$ | Cl | H | H | $-CH_2OCH_3$ | |

Tabelle 2:

Zwischenprodukte der Formel

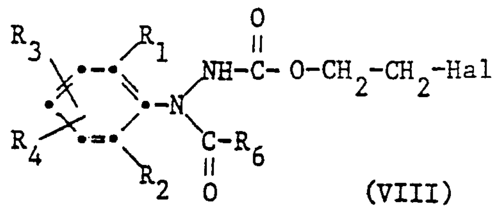

(VIII)

| Verb. Nr. | $R_1$ | $R_2$ | $R_3$ | $R_4$ | $R_6$ | Hal | Physik. Konst. |
|---|---|---|---|---|---|---|---|
| 2.1 | $CH_3$ | $CH_3$ | H | H | $-CH_2OCH_3$ | Br | Oel |
| 2.2 | $CH_3$ | $CH_3$ | H | H | Cyclopropyl | Br | |
| 2.3 | $CH_3$ | $CH_3$ | H | H | $-CH_2Cl$ | Br | Oel |
| 2.4 | $CH_3$ | $CH_3$ | H | H | 2-Tetrahydrofuryl | Br | |
| 2.5 | $CH_3$ | $CH_3$ | H | H | 2-Furyl | Br | |
| 2.6 | $CH_3$ | $CH_3$ | $3-CH_3$ | H | $-CH_2OCH_3$ | Br | |
| 2.7 | $CH_3$ | $CH_3$ | $3-CH_3$ | H | Cyclopropyl | Br | |
| 2.8 | $CH_3$ | Cl | H | H | $-CH_2OCH_3$ | Br | Smp.127-129°C |
| 2.9 | $CH_3$ | Cl | H | H | Cyclopropyl | Br | |
| 2.10 | $CH_3$ | Cl | H | H | $-CH_2Cl$ | Br | |
| 2.11 | $OCH_3$ | $CH_3$ | H | H | $-CH_2OCH_3$ | Br | |
| 2.12 | $CH_3$ | $NO_2$ | H | H | $-CH_2OCH_3$ | Br | |
| 2.13 | $OCH_3$ | Cl | H | H | $-CH_2OCH_3$ | Br | |
| 2.14 | $CH_3$ | $CH_3$ | 3-Cl | H | $-CH_2OCH_3$ | Br | |
| 2.15 | $CH_3$ | $CH_3$ | 3-Cl | H | Cyclopropyl | Br | |

Formulierungsbeispiele

Beispiel 2:

Feste Aufarbeitungsformen:

Stäube- und Streumittel enthalten im allgemeinen bis zu 10% des Wirkstoffs. Ein 5%-iges Stäubemittel kann beispielsweise aus 5 Teilen des Wirkstoffs und 95 Teilen eines Zuschlagstoffes wie Talkum bestehen oder aus 2 Teilen Wirkstoff, 1 Teil hochdisperser Kieselsäure und 97 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

Granulate wie Umhüllungsgranulate, Imprägniergranulate, Homogengranulate und Pellets [=Körner] enthalten üblicherweise 1 bis 80% des Wirkstoffs. So kann sich ein 5%-iges Granulat z.B. aus 5 Teilen des Wirkstoffs, 0,25 Teilen epoxidierten Pflanzenöles, 0,25 Teilen Cetylpolyglykolether, 3,50 Teilen Polyethylenglykol und 91 Teilen Kaolin (bevorzugt Korngrösse 0,3-0,8 mm) zusammensetzen. Man kann bei der Herstellung des Granulates wie folgt vorgehen:

Die Aktivsubstanz wird mit epoxidiertem Pflanzenöl vermischt und mit 6 Teilen Aceton gelöst, hierauf wird Polyethylenglykol und Cetylpolyglykolether zugesetzt. Die so erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 3:

Man unterscheidet im allgemeinen zwischen Wirkstoffkonzentraten, die in Wasser dispergierbar oder löslich sind und Aerosolen. Zu den in Wasser dispergierbaren Wirkstoffkonzentraten zählen z.B. Spritzpulver (wettable powders) und Pasten, die üblicherweise in den Handelspackungen 25-90% und in gebrauchsfertigen Lösungen 0,01 bis 15% des Wirkstoffs enthalten. Emulsionskonzentrate enthalten 10 bis 50% und Lösungskonzentrate enthalten in der gebrauchsfertigen Lösung 0,001 bis 20% Aktivsubstanz. So kann ein 70%-iges Spritzpulver z.B. aus 70 Teilen des Wirkstoffs, 5 Teilen Natriumdibutylnaphthylsulfonat, dazu 3 Teilen Naphthalinsulfonsäuren - Phenolsulfonsäuren - Formaldehyd-Kondensat (im Mischverhältnis 3:2:1), 10 Teilen Kaolin und 12 Teilen Kreide z.B. Champagne-Kreide zusammengesetzt sein. Ein 40%-iges Spritzpulver kann z.B. aus folgenden Stoffen bestehen: 40 Teile Wirkstoff, 5 Teile Natrium-Ligninsulfonat, 1 Teil Natrium-Dibutylnaphthylsulfonat und 54 Teile Kieselsäure. Die Herstellung eines 25%-igen Spritzpulvers kann auf unterschiedliche Art erfolgen. So kann dieses sich z.B. zusammensetzen aus: 25 Teilen der Aktivsubstanz, 4,5 Teilen Calcium-Ligninsulfonat, 1,9 Teilen Kreide, z.B. Champagne-Kreide/Hydroxyethylencellulose-Gemisch (1:1), 1,5 Teilen Natrium-Dibutylnaphthylsulfonat, 19,5 Teilen Kieselsäure, 19,5 Teilen (Champagne)-Kreide und 28,1 Teilen Kaolin. Ein 25%-iges Spritzpulver kann z.B. auch bestehen aus 25 Teilen Wirkstoff, 2,5 Teilen Isooctylphenoxypolyoxyethylen-ethanol, 1,7 Teilen (Champagne)-Kreide/Hydroxyethylencellulosegemisch (1:1), 8,3 Teilen Natriumsilikat, 16,5 Teilen Kieselgur und 46 Teilen Kaolin. Ein 10%-iges Spritzpulver lässt sich z.B. herstellen aus 10 Teilen des Wirkstoffes, 3 Teilen eines Gemisches aus Natriumsalzen von gesättigten Fettalkoholsulfonaten, 5 Teilen Naphthalinsulfonsäure/Formaldehyd-Kondensat und 82 Teilen Kaolin. Andere Spritzpulver können Gemische darstellen aus 5 bis 30% der Aktivsubstanz zusammen mit 5 Teilen eines aufsaugenden Trägermaterials wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykolethers. Ein 25%-iges Emulsions-Konzentrat kann z.B. folgende emulgierbare Stoffe enthalten: 25 Teile des Wirkstoffes, 2,5 Teile epoxidiertes Pflanzenöl, 10 Teile eines Alkylarylsulfonat-Fettalkoholpolyglykolether-Gemisches, 5 Teile Dimethylformamid und 57,5 Teile Xylol.

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Blattapplikation verwenden lassen. Solche Mittel gehören ebenfalls zur Erfindung.

Mittel, die wie oben beschrieben, als Wirkkomponente eine Verbindung der Formel I enthalten, beispielsweise Verbindung Nr. 1.1, 1.8 oder 1.24 lassen sich mit gutem Erfolg gegen schädliche Mikroorganismen einsetzen, insbesondere gegen blatt-, boden- und samenbürtige Pilze, wenn sie beispielsweise in Form eines Spritzmittels appliziert werden.

Mit gleich gutem oder ähnlichem Erfolg lassen sich auch Mittel einsetzen, die als Wirkstoff eine der übrigen Substanzen aus den Tabellen 1 und 2 enthalten.

Biologische Beispiele:

Beispiel 4: Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

b) Residual-kurative Wirkung

Tomatenpflanzen wurden nach 3-wöchiger Anzucht mit einer Sporangiensuspension des Pilzes infiziert. Nach einer Inkubation von 22 Stunden in einer Feuchtkammer bei 90-100% relativer Luftfeuchtigkeit und 20°C wurden die infizierten Pflanzen getrocknet und mit einer aus Spritzpulver des Wirkstoffes hergestellten Spritzbrühe (0,06% Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

c) Systemische Wirkung

Zu Tomatenpflanzen wurde nach 3-wöchiger Anzucht eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

Unter anderem verhinderten in obigen Versuchen a+b die Verbindungen Nr. 1.1, 1.8, 1.24 und 2.8 im Vergleich zu unbehandelten Kontrollpflanzen (=100% Befall) den Pilzbefall fast vollständig. Darüberhinaus zeigten die Verbindungen Nr. 1.1, 1.24 und 2.8 eine sehr gute systemische Wirkung.

Beispiel 5: Wirkung gegen Pythium debaryanum auf Rüben; Wirkung nach Bodenapplikation

Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussaat wurden die als Spritzpulver formulierten Versuchspräparate als wässrige Suspensionen über die Erde gegossen (20 ppm einer der Verbindungen aus den Tabellen 1 und 2 bezogen auf das Erdvolumen). Die Töpfe wurden darauf während 2-3 Wochen im Gewächshaus bei ca. 20°C aufgestellt. Die Erde wurde dabei durch leichtes Ueberbrausen stets gleichmässig feucht gehalten. Bei der Auswertung des Tests wurde der Auflauf der Zuckerrübenpflanzen sowie der Anteil gesunder und kranker Pflanzen bestimmt.

Die Verbindungen Nr. 1.1 bis 1.45 erwiesen volle Wirksamkeit, d.h. über 80% der Pflanzen liefen gesund auf.

Unter anderem zeigten die Verbindungen Nr. 1.1, 1.24 und 2.8 in obigen Versuchen eine sehr gute Wirkung gegen Pythium-Erreger auf Rüben. Eine gleich gute Wirkung wurde bei analogen Versuchen gegen Pythium-Erreger auf Mais, erzielt. Ueber 90% der Pflanzen liefen auf und zeigten ein gesundes Aussehen.

Beispiel 6: Wirkung gegen Xanthomonas oryzae auf Reis

Systemische Wirkung: Reispflanzen der Sorte "Caloro" oder "S6" wurden nach 3-wöchiger Anzucht im Gewächshaus mit einer Suspension der Prüfsubstanz begossen (0,006% Aktivsubstanz bezogen auf das Erdvolumen). Drei Tage nach dieser Behandlung wurden die Pflanzen in einem Klimaraum bei 24°C und 75-85% relativer Luftfeuchtigkeit aufgestellt und infiziert. Die Infektion erfolgte, indem die Blattspitzen mit einer Schere, die zuvor in eine Suspension von Xanthomonas oryzae eingetaucht worden war, abgeschnitten wurden. Nach 10-tägiger Inkubation in demselben Raum wurden die angeschnittenen Blätter welk, rollten sich ein und wurden nekrotisch. Das Ausmass dieser Krankheitssymptome diente zur Beurteilung der systemischen Wirksamkeit der Prüfsubstanz.

Pflanzen, die mit Verbindung Nr. 1.24 behandelt wurden, zeigten im Vergleich zu unbehandelten Kontrollpflanzen (100% Nekrose) weniger als 10% Nekrose.

Patentansprüche:

1. Verbindungen der Formel I

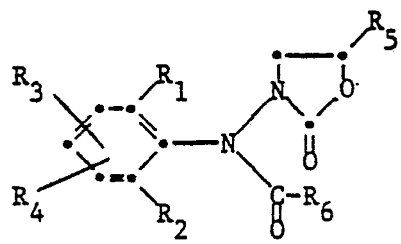

(I)

worin

$R_1$ $C_1$-$C_3$-Alkyl, $C_1$-$C_3$-Alkoxy oder Halogen,

$R_2$ $C_1$-$C_3$-Alkyl, Halogen oder Nitro,

$R_3$ Wasserstoff, Halogen, Nitro oder Methyl,

$R_4$ Wasserstoff oder Methyl und

$R_6$ für $-CH_2OR_7$, 1H-1,2,4-Triazolylmethyl, Halogenmethyl, Cyclopropyl, Furyl, Tetrahydrofuryl, $-CH_2N(C_1$-$C_3$-Alkyl$)_2$, $-CH_2NHN(R_8)(R_9)$ oder $-CH_2S(O)_n(C_1$-$C_3$-Alkyl) steht, wobei

$R_7$ Wasserstoff, $-SO_2(C_1$-$C_3$-Alkyl), $-SO_2NHCH_3$, Acetyl oder $C_1$-$C_3$-Alkyl,

$R_8$ Wasserstoff oder $C_1$-$C_3$-Alkyl,

$R_9$ $C_1$-$C_3$-Alkyl oder Phenyl bedeuten und

n 0, 1 oder 2 darstellt.

2. Verbindungen der Formel I nach Anspruch 1, worin $R_1$ für $C_1$-$C_3$-Alkyl, $C_1$-$C_3$-Alkoxy oder Halogen, $R_2$ für $C_1$-$C_3$-Alkyl oder Halogen, $R_3$ für Wasserstoff, Methyl, Halogen oder Nitro, $R_4$ für Wasserstoff oder Methyl stehen und $R_6$ $-CH_2OR_7$, 1H-1,2,4-Triazolylmethyl, Chlormethyl, Tetrahydrofuryl oder $-CH_2S(C_1$-$C_3$ -Alkyl) bedeutet, wobei $R_7$ für $C_1$-$C_3$-Alkyl, $-SO_2(C_1$-$C_3$-Alkyl) oder $-SO_2NHCH_3$ steht.

3. Verbindungen der Formel I nach Anspruch 2, worin $R_6$ für $-CH_2OR_7$, 1H-1,2,4-Triazolylmethyl oder 2-Tetrahydrofuryl steht, wobei $R_7$ für $C_1$-$C_3$-Alkyl, $-SO_2(C_1$-$C_3$-Alkyl) oder $-SO_2NHCH_3$ steht.

4. Verbindungen der Formel I nach Anspruch 1, worin $R_1$ Methyl, Methoxy oder Chlor, $R_2$ Methyl oder Chlor, $R_3$ Wasserstoff, Methyl oder Chlor und $R_4$ Wasserstoff oder Methyl bedeuten und $R_6$ für $-CH_2OR_7$ steht, wobei $R_7$ $C_1$-$C_3$-Alkyl, $-SO_2CH_3$ oder $-SO_2NHCH_3$ bedeutet.

5. Die Verbindung 3-[(N-Methoxyacetyl)-2,6-dimethyl-phenylamino]-oxazolidin-2-on nach Anspruch 1.

6. Die Verbindung 3-[(N-Methoxyacetyl)-2-chlor-6-methyl-phenylamino]-oxazolidin-2-on nach Anspruch 1.

7. Verfahren zur Herstellung von Verbindungen der Formel I nach Anspruch 1, dadurch gekennzeichnet, dass man Verbindungen der Formel II

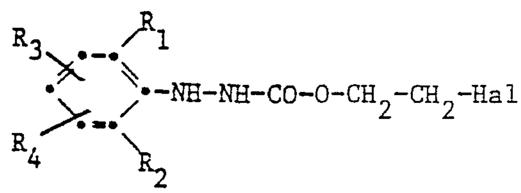

(II),

worin $R_1$, $R_2$, $R_3$ und $R_4$ die unter Formel I angegebenen Bedeutungen haben und Hal für Halogen steht, in der Reihenfolge wahlweise
a) mit einer Säure der Formel $HOOCR_6$ bzw. einem ihrer reaktionsfähigen Derivate acyliert, wobei $R_6$ die unter Formel I angegebenen Bedeutungen hat und b) in alkalischem Medium durch intramolekulare Halogenwasserstoff-Eliminierung cyclisiert und, falls $R_6$ für Halogenmethyl steht, die erhaltenen Verbindungen, sofern gewünscht, wahlweise mit einer der Verbindungen der Formel III, IV, V oder VI

$$HN(C_1\text{-}C_3\text{-Alkyl})_2 \qquad (III)$$

$$H_2N\text{-}N(R_8)(R_9) \qquad (IV)$$

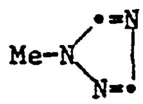 (V)

$MeOR_7$ (VI)

reagieren lässt, wobei $R_7$, $R_8$ und $R_9$ die unter Formel I angegebenen Bedeutungen haben und Me für Wasserstoff oder ein Metallkation steht.

8. Mittel zur Bekämpfung und/oder Verhütung eines Befalls durch Mikroorganismen, dadurch gekennzeichnet, dass es als mindestens eine aktive Komponente eine Verbindung der Formel I gemäss Anspruch 1 zusammen mit einem oder mit mehreren Trägerstoffen enthält.

9. Mittel nach Anspruch 8, dadurch gekennzeichnet, dass es als aktive Komponente eine Verbindung der Formel I gemäss einem der Ansprüche 2 bis 6 enthält.

10. Verwendung einer Verbindung der Formel I nach Anspruch 1 zur Bekämpfung und/oder Verhütung eines Befalls durch Mikroorganismen.

11. Verwendung nach Anspruch 10 von Verbindungen der Formel I gemäss einem der Ansprüche 2 bis 6.

12. Verwendung nach einem der Ansprüche 10 und 11, dadurch gekennzeichnet, dass es sich bei den Mikroorganismen um phytophathogene Pilze und/oder Bakterien handelt.

0030923

Europäisches Patentamt

**EUROPÄISCHER RECHERCHENBERICHT**

Nummer der Anmeldung

EP 80 ~~80~~ 0385

| Kategorie | EINSCHLÄGIGE DOKUMENTE<br>Kennzeichnung des Dokuments mit Angabe, soweit erforderlich der maßgeblichen Teile | betrifft Anspruch | KLASSIFIKATION DER ANMELDUNG (Int Cl ) |
|---|---|---|---|
| | US - A - 4 097 262 (JIIN-DUEY CHENG)<br>* Spalten 20-24; Ansprüche *<br>-- | 1,8 | C 07 D 263/26<br>413/12<br>A 01 N 43/76//<br>C 07 C 133/02 |
| D | JOURNAL OF THE ORGANIC CHEMISTRY, Band 31, Nr. 3, 11. März 1966, Washington, US,<br>M. HAUSER: "The preparation and cyclization of Chloroethyl Carbazates. Some Clarifications", Seiten 968-970<br>* Das ganze Dokument *<br>------ | 1,7 | |

RECHERCHIERTE SACHGEBIETE (Int Cl )

C 07 D 263/26
413/12
A 01 N 43/76

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 19-03-1981 | ALLARD |